Europäisches Patentamt

European Patent Office

Office européen des brevets

(19)

(11) Veröffentlichungsnummer: **0 156 806**
**B1**

(12) **EUROPÄISCHE PATENTSCHRIFT**

(45) Veröffentlichungstag der Patentschrift:
09.03.88

(51) Int. Cl.⁴: **A 61 F 2/10,** A 61 B 17/00

(21) Anmeldenummer: 84900044.3

(22) Anmeldetag: 09.12.83

(86) Internationale Anmeldenummer:
PCT/EP 83/00329

(87) Internationale Veröffentlichungsnummer:
WO 84/02464 (05.07.84 Gazette 84/16)

(54) **VERFAHREN ZUR HERSTELLUNG EINES TRANSPLANTATS; SCHNEIDVORRICHTUNG UND STANZVORRICHTUNG ZUR DURCHFÜHRUNG DES VERFAHRENS.**

(30) Priorität: 22.12.82 DE 3247387

(43) Veröffentlichungstag der Anmeldung:
09.10.85 Patentblatt 85/41

(45) Bekanntmachung des Hinweises auf die Patenterteilung:
09.03.88 Patentblatt 88/10

(84) Benannte Vertragsstaaten:
AT BE CH DE FR GB LI LU NL SE

(56) Entgegenhaltungen:
DE - B - 1 267 784
US - A - 3 076 461

Plastic and Reconstructive Surgery, August 1982, C.C. Yang et al.:"A Chinese Concept of Treatment of Extensive Third-Degree Burns", Seiten 238-252

(73) Patentinhaber: HETTICH, Rolf, Bohnenberger Strasse 5, S-7400 Tübingen (DE)

(72) Erfinder: HETTICH, Rolf, Bohnenberger Strasse 5, S-7400 Tübingen (DE)

(74) Vertreter: Dreiss, Uwe, Dr. jur. Dipl.-Ing. M.Sc. et al, Patentanwälte Dreiss, Hosenthien & Fuhlendorf Gerokstrasse 6, D-7000 Stuttgart 1 (DE)

## Beschreibung

Die Erfindung betrifft ein Verfahren zur Herstellung eines Transplantats, bestehend aus mit Öffnungen versehener Fremdhaut und in die Öffnungen eingesetzten Eigenhautstückchen.

Bei schweren Verbrennungen besteht das Problem der Defektdeckung darin, dass oft nur noch sehr geringe Fläche der intakten Eigenhaut des Patienten (z.B. am Kopf) für eine Transplantatentnahme zur Verfügung stehen. Eine endgültige Deckung von Hautdefekten mit Fremdhaut oder Hautersatzfolien allein ist jedoch bis heute nicht möglich. Die verfügbare Eigenhaut muss also möglichst ökonomisch eingesetzt werden. Man trägt die noch gesunde Eigenhaut oft mehrmals ab, um so nach und nach die verbrannten Flächen mit Transplantat zu versorgen.

Es sind auch Vorrichtungen bekannt, um Eigenhaut in viele kleine Eigenhautstückchen zu zerschneiden (US-A-3076461); man kennt ferner Vorrichtungen, um zur chirurgischen Korrektur von Haarausfall Hautstückchen bestimmter Grösse zwischen der Kopfhaut eines Spenders und der eines Empfängers auszuwechseln (DE-B-1267784).

Es ist ferner bekannt, dass nicht die gesamten Flächen, die durch eine Verbrennung beschädigt worden sind, durch Eigenhaut besetzt werden müssen. Es reicht aus, wenn man die durch Verbrennung beschädigten Bereiche mit körperfremdem Gewebe, z.B. mit Leichenhaut, abdeckt und nur in gewissen Abständen Inseln aus Eigenhaut einpflanzt. Aus diesen Inseln wachsen körpereigene Epithelzellen über das körperfremde Corium oder synthetische Coriumersatzfolien aus und bilden so einen definitiven kosmetisch funktionell voll befriedigenden Verschluss der Verbrennungsdefekte. Siehe Yang Chih-chun, Shih Tsi-siang, and Xu Wei-shia, A Chinese Concept of Treatment of Extensive Third-Degree Burns, Plastic and Reconstructive Surgery, August 1982, S.238–252.

Man hat seither nur von Hand in einem sehr mühsamen Verfahren in Leichenhautteile kleine runde Öffnungen eingeschnitten und in diese dann entsprechende Eigenhautstückchen eingesetzt. Dies ist ausserordentlich zeitraubend. Man benötigt selbst bei einer Gruppe von mehreren Operateuren für die Aufbereitung der Transplantate, die aus Fremdhaut und eingepflanzten Eigenhautstückchen gebildet werden, z.B. für die Deckung eines einzigen Armes mehrere Stunden.

Es ist Aufgabe der vorliegenden Erfindung, ganz allgemein Mittel bereitzustellen, um diesen Vorgang zu präzisieren, zu beschleunigen und zu vereinfachen. Erfindungsgemäss geschieht das durch das in dem Patentanspruch 1 angegebenen Verfahren und dessen vorteilhafte Weiterbildungen, vorzugsweise mit Hilfe der in den Patentansprüchen 7 u.ff. genannten Geräte.

Auf diese Weise ist es möglich, sehr schnell exakt definierte quadratische Eigenhautstückchen zu schneiden und andererseits entsprechende quadratische Öffnungen in die Leichenhaut einzuschneiden und die Eigenhautstückchen in diese Öffnungen in der Leichenhaut umzusetzen. Dies muss auch nicht notwendigerweise am Operationstisch geschehen; es ist möglich, die Vorbereitung dieser Transplantate in einem separaten Arbeitsgang labormässig vorzubereiten, sobald die erforderliche Eigenhaut zur Verfügung steht.

Das beschriebene Verfahren bzw. die beschriebenen Einrichtungen sind besonders deshalb sehr zweckmässig, weil die Haut des Menschen ausserordentlich zäh und schwer zu handhaben ist. Kleine Hautstückchen mit Pinzetten zu manipulieren, ist u.a. besonders deshalb sehr schwierig, weil die Haut die Tendenz hat, sich zusammenzurollen, an jeglicher Art von manipulierendem Werkzeug (wie z.B. Pinzette) zu kleben und dgl.

Die Erfindung gewährleistet ferner eine 100%ige Ausnutzung der noch verfügbaren Eigenhaut. Als «Fremdhaut» kommt sowohl Leichenhaut, tierische Hautteile sowie Hautersatzpräparate auf synthetischer Grundlage in Betracht.

Ausführungsbeispiele der Erfindung und ihrer vorteilhaften Weiterbildungen werden im folgenden anhand der beigefügten Zeichnungen beschrieben. Es stellen dar:

Fig. 1 einen Querschnitt eines Transplantats;

Fig. 2 eine Draufsicht auf für die Herstellung eines Transplantats vorbereitete Leichenhaut;

Fig. 3 eine Vorrichtung zum Schneiden von Eigenhaut;

Fig. 4 die Vorrichtung nach Fig. 3 um 90° versetzt;

Fig. 5 einen Querschnitt entlang der Linie V–V in Fig. 3;

Fig. 6 einen Querschnitt entlang der Linie VI–VI in Fig. 3;

Fig. 7 eine Modifikation der Schneidvorrichtung nach den Fig. 3 bis 6;

Fig. 8 einen Schnitt entlang der Linie VIII–VIII in Fig. 7;

Fig. 9 einen Schnitt durch die in der Schneidvorrichtung nach den Fig. 3 bis 6 verwendeten Plättchen 20 mit aufgesetztem Transportaufsatz 51;

Fig. 10 die Draufsicht auf eine Stanzeinrichtung zum Ausstanzen von Öffnungen in der Leichenhaut;

Fig. 11 einen Schnitt entlang der Linie XI–XI in Fig. 10;

Fig. 12 eine Draufsicht auf eine Umsetzeinrichtung 90.

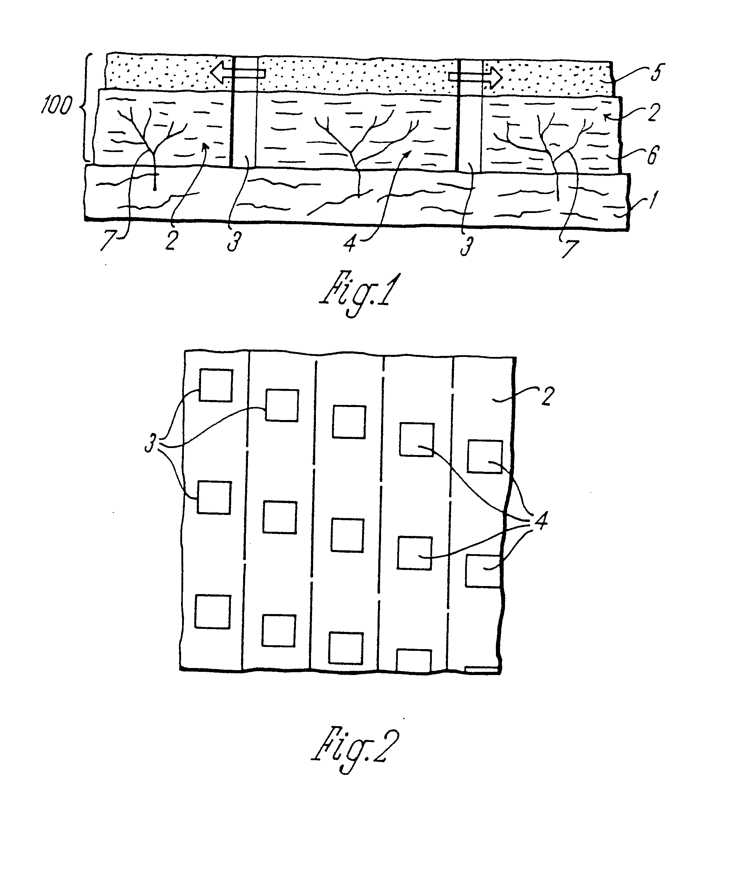

Nach Fig. 1 ist ein Transplantat im Zuge einer Hauttransplantation auf intaktes Gewebe 1 aufgesetzt. Da in vielen Fällen nicht – wie das an sich wünschenswert wäre – genügend Eigenhaut eines Patienten zur Verfügung steht, z.B. bei grossflächigen Verbrennungen, nimmt man grossflächige Stücke Fremdhaut 2 (Fig. 2) und deckt damit das Gewebe 1 ab; in der Fremdhaut 2 sind Öffnungen 3 vorgesehen, in die kleine Eigenhautstückchen eingesetzt werden.

Haut besteht, stark vereinfacht, aus Epithel oder Epidermis 5 (Oberhaut) und Corium 6 (Lederhaut). Das Epithel 5 wird wegen seines hohen Antigengehaltes (der Stimulationsindex einer gemischten Epithel-Lymphocytenkultur ist etwa 500 mal so hoch wie der einer gemischten fibroplastischen Lymphocytenkultur, das entspricht den Bausteinen des Coriums von Fremdhaut) vom Immunsystem sehr schnell als fremdes Gewebe erkannt und abgestossen; gleichzeitig wächst das Epithel der Eigenhautstückchen 4 in Pfeilrichtung auf das Corium der Fremdhaut 2 aus; währenddessen sprossen Gefässe 7 vom intakten Gewebe 1 in das Corium 6 ein. Man spricht von einem «Sandwich-Phänomen» und meint damit, dass nach dem Ersatz des Epithels 5 der Leichenhaut 2 durch aus den Eigenhautstückchen 4 gewachsenes neues Epithel das fremde Corium zwischen dem neu aufgebauten körpereigenen Epithel und eigenem intaktem Gewebe liegt, bis es nach und nach resorbiert und durch körpereigenes Gewebe ersetzt wird.

Ein Transplantationsverfahren dieser Art ist ungeheuer zeitaufwendig. Der Hauptgrund ist, dass Haut infolge ihrer natürlichen Eigenschaften sehr schwer zu handhaben ist; sie ist sehr widerstandsfähig; das bedeutet zunächst, das sie sehr schwer präzise zu schneiden ist. Ausserdem sind Hautstücke – wie sie etwa zum Zwecke der Transplantation mit einem entsprechenden Gerät (Dermatom) von gesunden Körperbereichen abgetragen werden – schwer manipulierbar; sie rollen und kleben zusammen; sie kleben ferner an Instrumenten (wie z.B. Pinzetten). Das Zusammensetzen eines grösseren Bereiches in der Art, wie es oben geschildert worden ist, ist selbst für ein aus mehreren Operateuren bestehendes Team extrem zeitraubend. Andererseits ist dies bei Schwerstverbrennungen der einzige Weg, mit nur wenig zur Verfügung stehender gesunder Eigenhaut die erforderlichen Flächen abdecken zu können.

Das erfindungsgemässe Verfahren bzw. die dazu geschaffenen Vorrichtungen bringen zu diesem Zweck erhebliche Vereinfachungen. Zunächst werden die einzelnen apparativen Komponenten beschrieben:

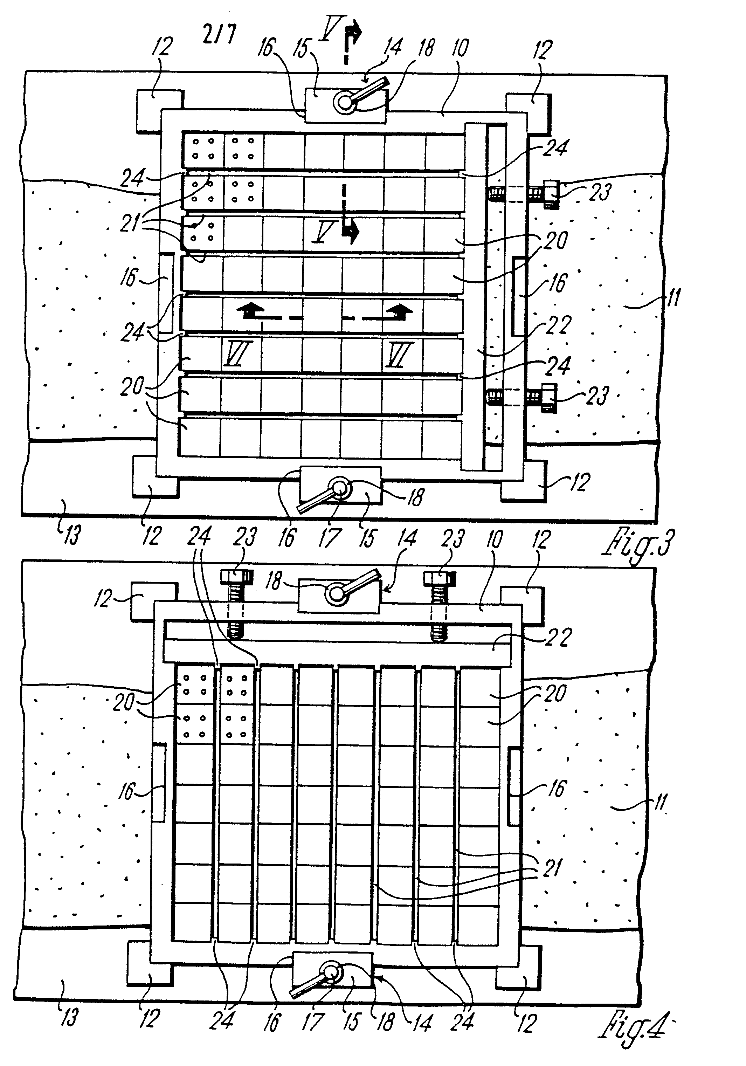

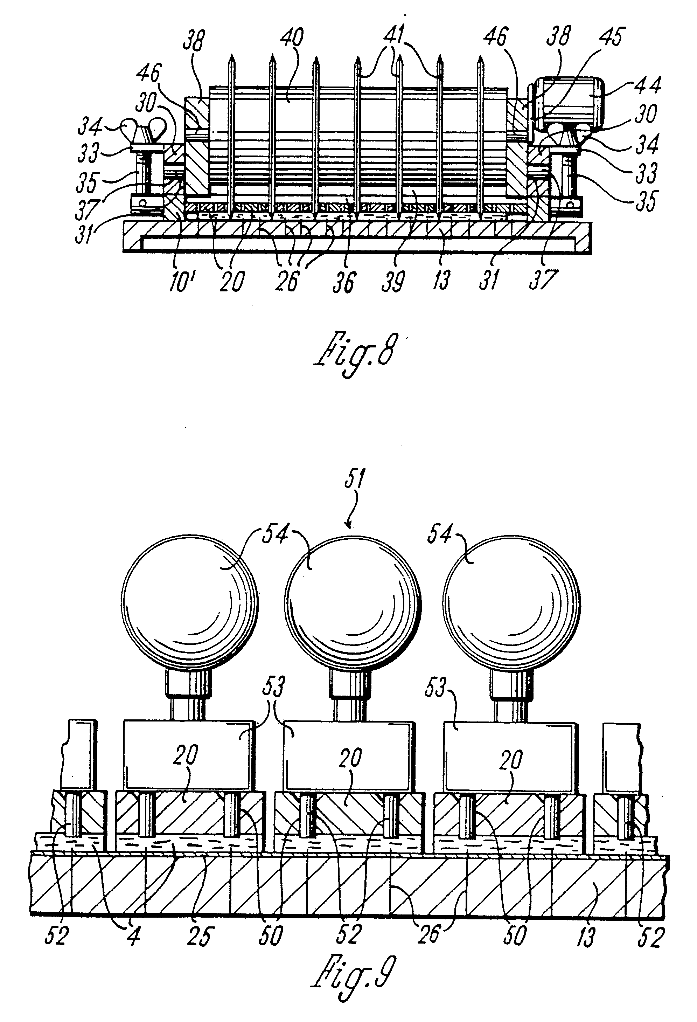

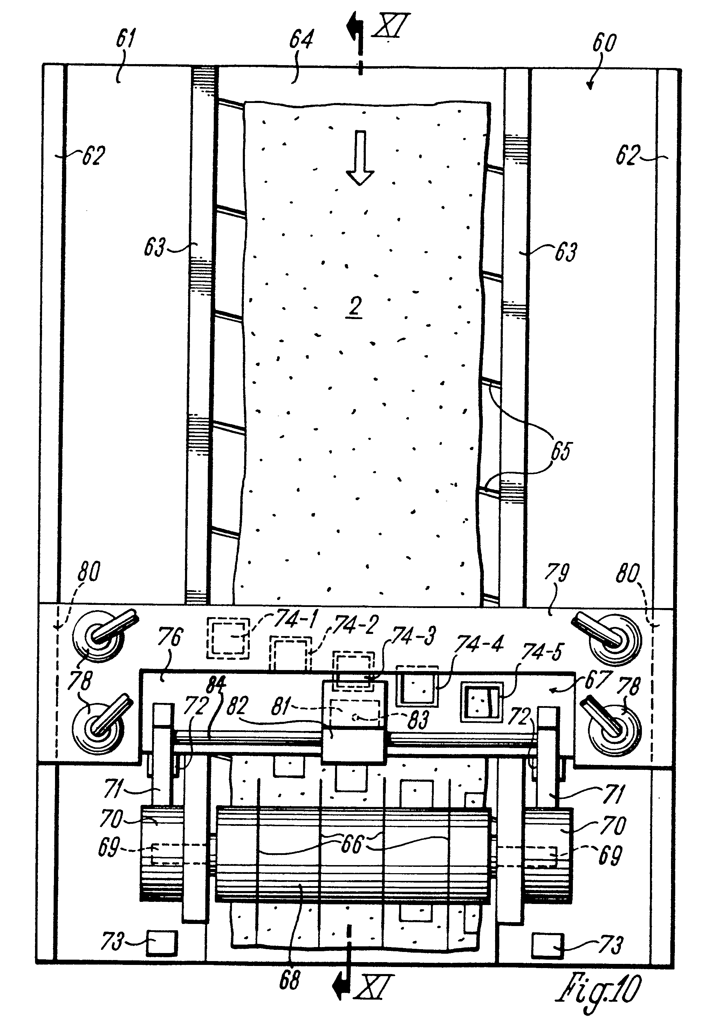

Der Schneiderahmen 10, wie er in den Fig. 3–6 und in einer Modifikation in den Fig. 7 und 8 dargestellt ist, dient dazu, Eigenhaut 11 in kleine rechteckige, vorzugsweise quadratische, Stückchen zu zerschneiden. Diese Eigenhautstückchen entsprechen dann dem Eigenhautstückchen 4 nach Fig. 1.

Der Schneiderahmen 10 ist mit den äusseren Abmassen quadratisch ausgebildet, so dass er in zwei Positionen, die gegeneinander um 90° verdreht sind (siehe Fig. 3 und 4) zwischen Positionierungsklötze 12 auf einer Grundplatte 13 eingesetzt werden kann. Zwischen Schneiderahmen 10 und Grundplatte 13 wird die in Eigenhautstückchen zu schneidende Eigenhaut 11 gelegt. Das Festspannen des Schneiderahmens 10 auf der Grundplatte 13 erfolgt mit Hilfe von Spanneinrichtungen 14. Eine Spanneinrichtung 14 (vgl. Fig. 5) besteht aus einem L-förmigen Winkelstück 15, das in Aussparungen 16 am Schneiderahmen 10 eingreift, einer Spannschraube 17 mit einem Andrückbund 18 und einer das Winkelstück 15 nach oben drückenden Feder 19. Mit Hilfe der Spanneinrichtungen 14 und der Positionierungsklötze 12 kann der Schneiderahmen 10 über der Eigenhaut 11 in eine genau definierte Position gebracht und in dieser festgespannt werden; dabei ist zwischen Schneiderahmen 10 und Grundplatte 13 die Eigenhaut 11 fest eingespannt.

In den Schneiderahmen 10 sind nur reihenweise quadratische Metallplättchen 20 eingespannt, und zwar derart, dass zwischen diesen Reihen Zwischenräume 21 entstehen. Sie dienen zum streifenweisen Schneiden der Eigenhaut 11. Diese Zwischenräume verlaufen in Fig. 3 waagrecht und in Fig. 4 – nach einer 90°-Umsetzung des Schneiderahmens 10 samt eingespannten Metallplättchen – senkrecht. Entlang dieser Zwischenräume 21 kann die Eigenhaut 11 zunächst waagrecht (Fig. 3) und dann senkrecht (Fig. 4) und somit in quadratische Eigenhautstückchen 4 geschnitten werden.

Um die Metallplättchen 20 in dem Schneiderahmen 10 in der dargestellten Weise einspannen zu können, ist im Schneiderahmen 10 in Fig. 3 auf der rechten Seite und in Fig. 4 oben verschiebbar eine Spannleiste 22 angeordnet. Sie wird durch Schrauben 23, die in Gewinden im Spannrahmen 10 aufgenommen sind, gegen die eingelegten Metallplättchen 20 gedrückt. Ersatzweise kann man vorsehen, dass ein exzentrischer Spannhebel die Spannleiste 22 an mehreren Punkten in der gewünschten Kompressionsstellung hält.

Die reihenweise Ordnung der Metallplättchen 20 im Schneiderahmen 10 derart, dass die Zwischenräume 21 entstehen, wird erleichtert durch Vorsprünge 24 im Schneiderahmen 10 bzw. in der Spannleiste 22. Das Einlegen der Metallplättchen 20 und das Spannen durch Anziehen der Schrauben 23 erfolgt auf einer glatten Unterlage, wobei zur geraden Ausrichtung der Plättchen und damit zur geraden Ausbildung der Zwischenräume 21 eventuell Abstandsleisten eingelegt werden können, die man nach dem Spannen wieder herausnimmt. Auch die Verwendung vorgeformter Plastikschablonen ist möglich.

Man spannt also zunächst den Schneiderahmen 10 in der in Fig. 3 dargestellten Weise ein. Daraufhin schneidet man entlang der Zwischenräume 21 die eingespannte Eigenhaut 11. Dann werden die Spanneinrichtungen 14 gelöst und der Schneiderahmen 10 um 90° gedreht, wieder eingesetzt und die Spanneinrichtungen 14 erneut angezogen. Dann erfolgen entlang der Zwischenräume 21 in Fig. 4 die senkrechten Schnitte. Damit ist die Eigenhaut 11 in eine Vielzahl quadratischer Eigenhautstückchen 4 geschnitten.

Die Metallplättchen 20 haben eine Kantenlänge von beispielsweise 5–10 mm. Eine entsprechende Grösse haben dann auch die quadratischen Eigenhautstückchen 4. Der Spannrahmen kann z.B. eine Kantenlänge von 25–30 cm haben und für verschieden grosse Plättchen 20 verwendet werden, entsprechend verschiedener Spannleisten 22.

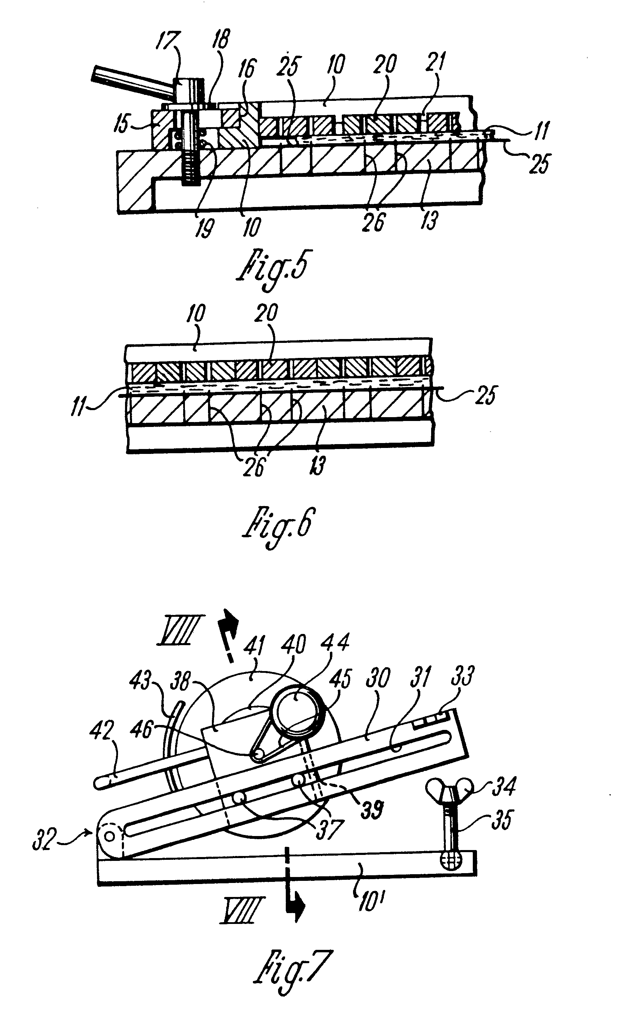

Wie aus Fig. 5 und 6 ersichtlich, liegt unter der Eigenhaut 11 eine Schneidefolie 25. Durch sie wird es erleichtert, die Eigenhaut 11 vollkommen und glattflächig durchzuschneiden, ohne dass das hierzu verwendete Skalpell oder ein sonstiges Schneidinstrument dabei direkt auf dem Metall der Grundplatte 13 unter Druck aufsetzen muss.

In der Grundplatte 13 sind nun, wie aus Fig. 5 und 6 ersichtlich, im Abstand von 2–3 mm Stahlstifte (26) eingelassen. Dies erfolgt derart, dass sie aus der Oberseite der Grundplatte 13 um so viel herausragen, dass sie gerade noch die Schneidefolie 25 durchstossen und mit der aus der Schneidefolie 25 um 0,1–0,2 mm herausragenden Spitze in die aufliegende Fläche der Eigenhaut 11 eindringen und diese auf der Grundplatte 13 festhalten, so dass sie sich nicht verschieben kann; insbesondere wird so vermieden, dass sich beim Schneiden und nach dem Abnehmen des Schneiderahmens 10 (siehe dazu weiter unten) die Eigenhautstückchen 4 wieder aufrollen oder eine unerwünschte Lage einnehmen. Die Spitzen der Stahlstifte 26 dringen somit nur knapp in die auf der Schneidefolie 25 aufliegenden Seite der Eigenhaut 11 ein. Die Eigenhaut 11 ist etwa 0,5–0,7 mm dick.

Es ist natürlich auch möglich, das Festhalten der Eigenhaut 11 auf der Oberfläche der Grundplatte 13 bzw. auf der Schneidefolie 25 mit anderen Mitteln zu bewirken, also durch entsprechende Vorsprünge oder anderweitig griffige Gestaltung der Oberfläche der Schneidfolie 25. Es wäre auch denkbar, die Eigenhaut mit – ggf. körperverträglichen Klebstoffen – auf die Grundplatte 13 aufzukleben. Allerdings müsste diese Verbindung dann wieder nach dem Schneiden gelöst werden.

Das Schneiden der Eigenhaut 11 bei eingespanntem Schneiderahmen kann im einfachen Fall mit einem Skalpell erfolgen. Man kann dies jedoch apparativ auch weiter vereinfachen. Dem dient die Modifikation nach Fig. 7 und 8:

In Fig. 7 und 8 sind auf dem Schneiderahmen 10', hochklappbar zwei Schneidwalzenführungen 30 angeordnet. Sie sind in Form einer länglichen Schiene ausgebildet und weisen einen Führungsschlitz 31 auf. Sie sind an dem Schneiderahmen 10' an der einen Seite mit Hilfe eines Gelenkes 32 angelenkt. Auf der anderen Seite können die Schneidwalzenführungen 30 mit Hilfe gabelförmig ausgebildeter Haken 33, Flügelschrauben 34 und Gewindebolzen 35, die an dem Schneiderahmen 10' angelenkt sind, festgespannt werden. Die beiden Schneidwalzenführungen 30 bilden zusammen mit einer Rückplatte 36 einen hochklappbaren Rahmen.

In den Führungsschlitzen 31 der Schneidwalzenführungen 30 laufen Führungszapfen 37, die je an einer Platte 38 angebracht sind. Die Rückplatte 39 verbindet die Platten 38 und ist zu diesen quer angeordnet. Derart bilden die Platten 38 zusammen mit der Rückplatte 39 einen Rahmen für eine Schneidwalze 40. Auf der Schneidwalze 40 sind in einem Abstand, der gleich dem Abstand der Zwischenräume 21 in Fig. 3 bzw. 4 ist, Schneidräder 41 angeordnet. An dem durch die beiden Platten 38 und die Rückplatte 39 gebildeten Rahmen ist ferner ein Handgriff 42 angebracht, der dazu dient, den Rahmen mit Schneidewalze 40 von dem einen Ende der Führungsschlitze 31 zu dem anderen Ende der Führungsschlitze 31 (also z.B. in Fig. 7 von rechts nach links) zu ziehen. Das Schutzblech 43 ist am Handgriff angebracht und dient zum Schutz der Hand. Die Schneidwalze 40 mit den Schneidrädern 41 wird von einem Motor 44 über Übertragungsriemen 45 und Welle 46 angetrieben. Die Welle 46 ist mit der Schneidwalze 40 fest verbunden. Die Schneidwalze 40 kann aus mehreren zylindrischen Abschnitten aufgebaut sein, die abwechselnd mit den Schneidrädern 41 auf die Welle 46 aufgesteckt sind, so dass das in Fig. 8 gezeigte Gebilde entsteht. Der Durchmesser der Schneidräder 41 ist so bemessen, dass beim Aufspannen dieses Schneidrahmens 10' auf die Grundplatte 13, wobei auf den Schneidrahmen 10' wiederum die Schneidwalzenführungen usw. aufgespannt sind, die Schneidräder gerade in die Schneidfolie 25 einschneiden, so dass die Eigenhaut 11 sicher durchtrennt wird.

Man kann anstelle der motorangetriebenen Schneidräder, die von Hand über die Schnittstrecke geschoben werden, auch andere mechanische Mittel zum Durchtrennen der Eigenhaut 11 vorsehen, so beispielsweise ein auf- und abbewegbares Stanzmesser oder dgl.

Nach Durchführung des Schneidvorganges mit einer Schneideinrichtung nach Fig. 3 bis 8 ist die Eigenhaut 11 in quadratische Eigenhautstückchen 4 geschnitten, die sich zunächst noch (vgl. Fig. 4) in einem zwischen den Metallplättchen 20 und der Grundplatte 13 eingespannten Zustand befinden. Die weitere Handhabung geschieht wie folgt:

Die Metallplättchen 20 sind in den dargestellten Ausführungsbeispielen mit vier zylindrischen Bohrungen 50 versehen. Dies ist in Fig. 9 besonders dargestellt. Sie ermöglichen es, auf die Metallplättchen 20 nach dem Schneiden der Eigenhaut 11 Druck/Saug-Einheiten 51 aufzusetzen. Es handelt sich dabei im weiteren Sinne um «Transportaufsätze»; sie haben den Zweck, die Eigenhautstückchen 4 zusammen mit den Metallplättchen 20 von der Unterlage, gebildet durch die Grundplatte 13, abzuheben und in die Öffnungen 3 in der Leichenhaut 2 (vgl. Fig. 2) einzusetzen.

Im Ausführungsbeispiel wird dieser Transportaufsatz durch vier Röhrchen 52 gebildet, die sich durch die vier Bohrungen 50 in einem Metallplättchen 20 hindurch erstrecken. Die Bohrungen 50 sind oben konisch, um das Einführen der Röhrchen zu erleichtern. Die Röhrchen 52 schliessen während der Transportphase auf ihrer Unterseite mit den Metallplättchen 20 ab, können aber beim Ablegen der Eigenhautstückchen leicht hervorstehen, um die Haut von den Plättchen 20 abzulösen und stehen auf ihrer Oberseite mit einer Kammer 53 in Verbindung, die mit einem zusammenpressbaren Gummiballon 54 in Verbindung steht.

Man muss nun nach dem Durchführen der Schnitte auf jedes der Metallplättchen 20 einen derartigen Transportaufsatz 51 aufsetzen und den Gummiballon 54 zusammendrücken. Dann sorgt der dadurch entstehende Unterdruck in den Röhrchen 52 dafür, dass beim Abheben des Transportaufsatzes von der Grundplatte 13 auch die Eigenhautstückchen 4 mit abgehoben werden und nicht auf der Grundplatte 13 hängen bleiben. Beim Abheben des Transportaufsatzes 51 wird also das Metallplättchen 20 und das darunter an ihm hängende Eigenhautstückchen 4 mit abgehoben. Der Unterdruck, der über die Röhrchen 52 an den Eigenhautstückchen 4 wirksam wird, braucht im Hinblick auf das äusserst geringe Gewicht eines derartigen Eigenhautstückchens 4 nur minimal zu sein. Diese Kraft reicht auch, um die Eigenhautstückchen 4 von den über die Oberfläche der Grundplatte 13 hervorragenden Spitzen der Stahlstifte 26 zu lösen, da die Spitzen wegen der Elastizität der Haut gar nicht in diese eindringen, sondern sie lediglich wie Krallen gegen eine Verschiebung auf der Grundplatte 13 sichern. Man kann vorsehen, dass die Röhrchen 52 in den Bohrungen 50 kein Spiel haben, so dass die Metallplättchen 20 durch Reibung der Röhrchen 52 in den Bohrungen 50 an den Transportaufsätzen gehalten werden.

Hat man nun die Transportaufsätze auf sämtliche Metallplättchen 20 aufgesetzt, so wird der Schneiderahmen zusammen mit den Metallplättchen 20, den Transportaufsätzen 51 und den unten anhängenden Eigenhautstückchen 4 in die Umsetzvorrichtung 90 (siehe Fig. 12) verbracht. Dann werden die Schrauben 23 im Schneiderahmen 10 gelöst, so dass die Transportaufsätze 51 zusammen mit dem darunter hängenden Metallplättchen 20 und die Eigenhautstückchen 4 einzeln weiter gehandhabt werden können und so in Öffnungen 3 in der Fremdhaut 2 eingesetzt werden können.

Man kann den Gummiballon 54 auch durch andere Einrichtungen ersetzen, die dazu dienen, in den Röhrchen 52 Unterdruck bzw. Überdruck zu erzeugen. Man kann z.B. alle Transportaufsätze über längere Leitungen mit einer gemeinsamen Einheit, die Unterdruck bzw. Überdruck erzeugt, verbinden. Da hier nur ganz kleine Druckdifferenzen notwendig sind, können die Leitungen so fein sein, dass sie nicht hinderlich sind. Die Umschaltung von Überdruck auf Unterdruck bzw. umgekehrt kann über Ventile erfolgen.

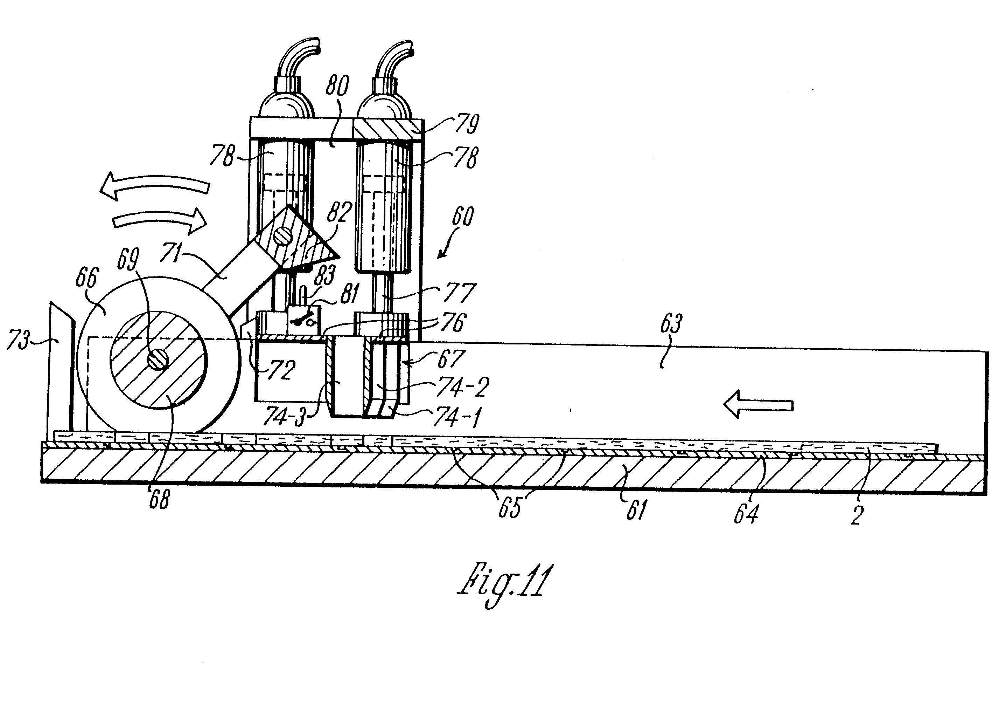

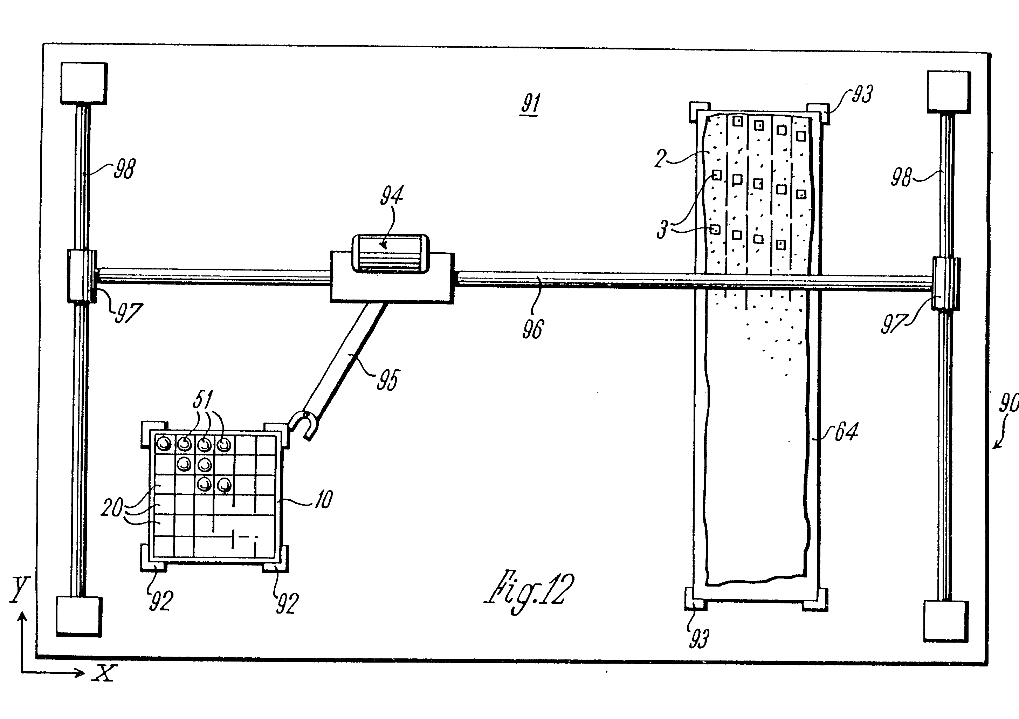

Zur Vorbereitung der Fremdhaut 2 für das Einsetzen von Eigenhautstückchen 4 in der Art und Weise, wie die Fremdhaut 2 in Fig. 12 dargestellt ist, dient eine Stanzeinrichtung 60 nach Fig. 10 und 11. Sie weist eine Grundplatte 61, zwei Seitenplatten 62, und zwei Führungsschienen 63 auf. Zwischen die Führungsschienen 63 wird die Fremdhaut 2 eingelegt. Sie liegt auf einer Transport- und Schnittfolie 64. Die Transport- und Schnittfolie 64 ist in an sich bekannter Weise mit schräg zur Transportrichtung verlaufenden Rillen versehen. Der Abstand der Rillen ist derart bemessen, dass jeweils zwischen zwei Rillen – ebenfalls schräg zur Transportrichtung verlaufend – eine Reihe Öffnungen 3 eingestanzt werden kann. Wird nun die Fremdhaut 2 unter den Schneidmessern 66 hindurchtransportiert, so schneiden die Schneidmesser 66 an dem Bereich, an der die Fremdhaut 2 zwischen den Rillen 65 plan auf der Transport- und Schnittfolie 64 aufliegt, durch die Haut hindurch. An den Stellen, an denen unter der Fremdhaut 2 sich die Rillen 65 befinden, kann die Haut vor dem Schnitt in die Rillen ausweichen. Zwischen den Einschnitten bleiben also über den Rillen schnittfreie Stege stehen, die die Haut zusammenhalten. Die auf diese Weise herbeigeführten Einschnitte ermöglichen nach der Transplantation, dass Sekret abfliessen kann, das sich zwischen Leichenhaut und Gewebe bildet. Dies ist an sich bekannt; die Verwendung im vorliegenden Zusammenhang ist jedoch besonders zweckmässig.

Wie aus Fig. 10 und 11 zu ersehen, wird die Transport- und Schnittfolie 64 in Transportrichtung (siehe Pfeil) unter einer Stanzeinheit 67 hindurchbewegt und durch eine Transport- und Schnittrolle 68, in der die Schneidmesser 66 angeordnet sind, sowohl geschnitten als auch taktweise durch Stanzen mit Öffnungen 3 versehen und gleichzeitig um eine genau definierte Strecke weiterbewegt.

Die Transport- und Schnittrolle 68 ist starr mit einer Achse 69 verbunden, die in den Führungsschienen 63 beidseitig gelagert ist. Die Achse 69 ist über eine Freilaufnabe 70 mit einem Handhebel 71 verbunden. Die Kopplung der Nabe 70 mit der Achse 69 ist derart, dass bei einer Bewegung des Handhebels 71 im Uhrzeigersinn (der untere Pfeil links in Fig. 11) bis zum Auftreffen auf einen Anschlag 72 sich die Achse 69 und mit ihr die Transport- und Schnittrolle 68 mit dem Handhebel 71 mitdreht; somit wird die Transport- und Schnittfolie 64 zusammen mit der Fremdhaut 2 zwischen Transport- und Schnittrolle 68 und Grundplatte 61 eingezogen und um ein genau definiertes Längenstück weiterbewegt. Daraufhin wird der Handhebel 71 wieder im Gegenuhrzeigersinn (der obere Pfeil links in Fig. 11) zurückbewegt bis zum Auftreffen auf den Anschlag 73. Bei dieser Bewegung im Gegenuhrzeigersinn bewegt sich die Achse 69 nicht mit dem Handhebel 71 mit. Sie dreht vielmehr frei durch. Eine solche Kupplung, durch die in der einen Drehrichtung eine bewegungsmässige Kopplung gegeben ist und in der anderen Drehrichtung nicht, ist an sich bekannt und bedarf daher keiner weiteren Erläuterung.

Bei der Transportbewegung, bei der in der bereits erwähnten Art und Weise die in Fig. 12 ersichtlichen Schnitte in die Fremdhaut eingeschnitten werden, läuft die Fremdhaut unter der Stanzeinheit 67 hindurch. Die Stanzeinheit 67 weist — schräg zur Transportrichtung und parallel zu den Rillen 65 — fünf Stanzwerkzeuge 74-1 bis 74-5 auf, die sämtliche quadratisch ausgebildet und unten als Schneide geschliffen sind. Bei einer Abwärtsbewegung auf die Fremdhaut 2 werden damit viereckige Löcher 3 in der in Fig. 12 dargestellten Konfiguration ausgeschnitten. Die fünf Stanzwerkzeuge 74-1 bis 74-5 sind an der Stanzplatte 76 befestigt. An dieser Stanzplatte greifen vier Kolben 77 an, die in Zylindern 78 geführt sind. Die Zylinder 78 sind an einer Kopfplatte 79 befestigt, die durch zwei Seitenplatten 80 gehalten wird. Die Seitenplatten 80 sind an den bereits erwähnten Seitenplatten 62 befestigt. Werden die Kolben 77 in den Zylindern 78 mit Druck beaufschlagt, so fährt die Stanzplatte 76 mit den Stanzwerkzeugen 74-1 bis 74-5 nach unten und stanzt eine Reihe von Öffnungen 3 aus der Fremdhaut 2 aus.

Die Synchronisierung des Stanzvorganges mit dem Transportvorgang erfolgt derart, dass auf der Stanzplatte 76 ein elektrischer Schalter 81 angeordnet ist, der betätigt wird, sobald der am Handhebel 71 angebrachte Anschlag 82 auf den Kontaktstift 83 des Schalters 81 auftrifft. Dies ist am Ende jedes Fortbewegungstaktes der Fall, der durch die Bewegung des Handhebels 71 im Uhrzeigersinn bis zum Auftreffen auf den Anschlag 72 definiert ist. Dann fährt das Stanzwerkzeug 67 (Stanzplatte 76 mit Stanzwerkzeugen 74-1 bis 74-5) nach unten, führt die Stanzung aus und fährt anschliessend wieder nach oben. Der Handhebel wird dann zurückbewegt.

Wie aus Fig. 10 ersichtlich, ist die Kopfplatte 79 in der Draufsicht U-förmig ausgebildet, so dass man mit der Hand gut die Querstange 84, die Bestandteil des Handhebels 71 ist und den Anschlag 83 trägt, ergreifen kann.

Ist nun die Schnitt- und Transportfolie 64 mit der aufgelegten Fremdhaut 2 vollkommen durch die Stanzeinrichtung 60 hindurchgelaufen, dann wird sie zusammen mit der aufliegenden, nunmehr geschnittenen Fremdhaut 2 ebenfalls in die Umsetzvorrichtung 90 nach Fig. 12 eingesetzt.

Die Umsetzvorrichtung 90 nach Fig. 12 besteht aus einer Platte 91, auf der erste Positionierungsklötze 92 zur Aufnahme des Schneiderahmens 10 (oder 10') und zweite Positionierungsklötze 93 zur Aufnahme der Transport- und Schnittfolie mit aufliegender Fremdhaut 2 angeordnet sind. Durch einen Manipulator 94 mit beweglichem Greifer 95, der sich in einem x/y-Koordinatensystem bewegt, werden nun die einzelnen Transportaufsätze 51 mit anhängendem Metallplättchen 20 und Eigenhautstückchen 4 nach Lösen der Schrauben 23 erfasst und nacheinander in die Öffnungen 3 in der Fremdhaut 2 eingesetzt. Der Manipulator 94 ist auf der Stange 96 in x-Richtung, die Stange 96 mit den beiden Muffen 97 auf den Stangen 98 in y-Richtung verschiebbar. Derartige programmierbare Manipulatoren sind im Handel erhältlich. Es ist daher nicht erforderlich, sie im vorliegenden Zusammenhang näher zu beschreiben; sie sind selbst nicht Gegenstand der Erfindung. Man muss sich vergegenwärtigen, dass die Lage der einzelnen Transportaufsätze 51 innerhalb des Schneiderahmens 10 und die Lage der Öffnungen 3 auf der Transport- und Schnittfolie 64 jeweils aufgrund der vorherigen maschinellen Bearbeitungsschritte genau definiert sind. Daher kann man den Manipulator 94 so programmieren, dass er einen Transportaufsatz 51 nach dem anderen in eine Öffnung 3 nach der anderen einsetzt. Es ist auch möglich, das Einsetzen ggf. ohne Vorgabe genauer Koordinaten der Öffnungen über Sensoren, die mit Lichtabtastung oder dgl. arbeiten zu steuern.

Nach dem Ablauf dieses Vorganges ist die Leichenhaut 2 mit Eigenhautstückchen 4 präpariert und das gesamte Transplantat, aus der Leichenhaut 2 mit eingesetzten Eigenhautstückchen 4 bestehend, kann von einem Operateur transplantiert werden.

Die Umsetzeinrichtung 90 kann auch anders ausgebildet sein. Eine vereinfachte Vorrichtung weist lediglich sowohl einerseits für den Schneiderahmen 10 als auch andererseits für die Transport- und Schnittfolie 64 Positionierungsklötze auf, in die diese Teile eingelegt werden können. In einer halbmechanisch ausgebildeten Form können die Transportaufsätze 51 auch von Hand in die einzelnen Öffnungen 3 in der Leichenhaut 2 umgesetzt werden.

Nachdem nun in der Umsatzeinrichtung 20 alle Eigenhautstückchen in die Fremdhaut 2 eingesetzt worden sind, wird das dadurch entstandene Transplantat mit einer Folie verklebt, die gewährleistet, dass das Transplantat beim Abnehmen von der Unterlage 64 seine Form beibehält, und dass dabei insbesondere die Eigenhautstückchen in den Öffnungen der Fremdhaut verbleiben. In dieser Form wird dann das Transplantat auf den abzudeckenden Teil eines Patienten verbracht.

Dabei ist es möglich, unter Zuhilfenahme der beschriebenen Vorrichtungen und Verfahren, die Herstellung des Transplantats sowohl räumlich als auch zeitlich von dem eigentlichen Operationsvorgang zu trennen; es wäre denkbar, die beschriebenen Einrichtungen mobil auszugestalten (in Spezialfahrzeugen oder dgl.) und mit einer entsprechend auf die Bedienung dieser Vorrichtungen geschulten Mannschaft jeweils kurzfristig an den Ort des Bedarfs zu bringen, also an ein Krankenhaus, in dem ein Patient mit schweren Verbrennungen zu versorgen ist.

Beim Patentanspruch 1 wird davon ausgegangen, dass die Transportaufsätze 51 zusammen mit den Metallplättchen 20 «Transportteile» bilden, d.h. Elemente, die es ermöglichen, die in dem Rahmen quadratisch geschnittenen Eigenhautstückchen in die Öffnungen 3 der Fremdhaut 2 zu verbringen, wobei der im Zusammenhang mit Fig. 12 beschriebene Greifer 95 die Transportaufsätze erfasst. Es ist auch möglich, diese Einrichtung dahingehend abzuwandeln, dass an dem Manipulator 94 bzw. am Ende des Greifers 95 spezielle Einrichtungen − etwa eine Zange mit Abstreifer oder dgl. − vorgesehen sind, die die Plättchen 20 zusammen mit den darunter liegenden Eigenhautstückchen erfassen, in die Öffnungen 3 der Fremdhaut 2 einsetzen und dort abgeben bzw. abstreifen. Dann brauchte man nicht so viele Transportaufsätze vorzusehen, wie Eigenhautstückchen vorgegeben sind; es würde vielmehr die Schaffung einer einzigen Einrichtung genügen.

Bei dem Transportaufsatz nach Fig. 9 ist vorgesehen, dass vier Röhrchen 50 sich nach unten durch Öffnungen in den Metallplättchen 20 hindurch erstrecken, über die beim Anheben Sog und beim Abgeben Druck auf die Eigenhautstückchen 4 ausgeübt wird. Es ist auch möglich, eine grössere Anzahl von Röhrchen vorzusehen, um ein Festkleben der Eigenhautstückchen 4 an der unteren Fläche der Metallplättchen 20 zu vermeiden. Im einzelnen können hier auch einfache Abstreifer oder ähnliche Mittel vorgesehen sein.

## Patentansprüche

1. Verfahren zur Herstellung eines Transplantats (100), bei welchem Eigenhautstückchen (4) vorgefertigt, an eine mit Öffnungen (3) versehene Fremdhaut (2) bewegt und in diese eingesetzt werden, dadurch gekennzeichnet, dass in eine Umsetzeinrichtung (90) an einer durch Positionierungsmittel (92) in einem Koordinatensystem definierten Stelle eine Halteeinrichtung (10) eingelegt wird, in der sich an ebenfalls definierten Stellen Eigenhautstückchen (4) befinden, auf die jeweils innerhalb der Halteeinrichtung (10) ein Transportteil (20, 51) aufgesetzt ist, der Mittel (52–54) aufweist, die beim Abheben das jeweilige Eigenhautstückchen (4) mitnehmen und beim Absetzen das Eigenhautstückchen abgeben, dass ferner an einer weiteren, innerhalb des Koordinatensystems durch weitere Positionierungsmittel (93) definierten Stelle eine Unterlage (64) mit darauf angeordneter Fremdhaut (2) eingelegt wird, in die – bezüglich der Unterlage (64) – an ebenfalls definierten Stellen die Öffnungen (3) eingeschnitten sind, und dass ferner ein Manipulator (94, 95) die Transportteile (20, 51) mit den Eigenhautstückchen (4) an die Öffnungen (3) in der Fremdhaut (2) bewegt und die Eigenhautstückchen (4) in diese einsetzt.

2. Verfahren nach Anspruch 1, dadurch gekennzeichnet, dass als Fremdhaut Leichenhaut, synthetische Hautersatzfolie oder sonstige Hautersatzpräparate eingesetzt werden.

3. Verfahren nach Anspruch 1, dadurch gekennzeichnet, dass in die Fremdhaut (2) Sekretabfluss-Schlitze (65) eingeschnitten sind.

4. Verfahren nach Anspruch 1, dadurch gekennzeichnet, dass die Eigenhautstückchen (4) vielekkig, insbesondere quadratisch oder sechseckig ausgebildet sind.

5. Verfahren nach Anspruch 4, dadurch gekennzeichnet, dass zur Herstellung quadratischer Eigenhautstückchen (4) entsprechend quadratisch ausgeformte Plättchen (20) reihenweise in der als Rahmen (10) ausgebildeten Halteeinrichtung mit geeigneten Spannmitteln (22, 23) derart eingespannt werden, dass sich zwischen den Reihen Zwischenräume (21) bilden, dass ferner der Rahmen (10) mit den eingespannten Plättchen (20) in einer ersten Position (Fig. 3) auf eine Unterlage (13) aufgesetzt und festgeklemmt wird, dass danach die Eigenhaut (11) entlang der Zwischenräume (21) geschnitten wird, darauf der Rahmen (10) mit der reihenweise eingespannten Plättchen (20) von der Eigenhaut (11) abgenommen, um 90° verdreht und erneut auf die Eigenhaut aufgesetzt und auf der Unterlage festgeklemmt wird, und danach die Eigenhaut wiederum entlang der Zwischenräume (21) geschnitten wird.

6. Verfahren nach Anspruch 5, dadurch gekennzeichnet, dass nach dem zweifachen Schneiden der Eigenhaut entlang der Zwischenräume (21) auf die Plättchen (20) Transportaufsätze (51) aufgesetzt werden, die zusammen mit den Plättchen (20) den gesamten Transportteil bilden.

7. Schneidvorrichtung zur Durchführung des Verfahrens nach Anspruch 5, mit der die Eigenhaut (11) in Stückchen (4) geschnitten wird, dadurch gekennzeichnet, dass auf einer Grundplatte (13) Spanneinrichtungen (14) zum Festspannen eines Rahmens (10) und Positionierungsmittel (12) vorgesehen sind, um dem Rahmen eine definierte Position zu geben, dass der Rahmen (10) um eine zur Grundplatte (13) senkrechte Achse drehbar angeordnet ist, und dass ferner längs einer Innenkante des Rahmens (10) und längs einer dieser Innenkante gegenüberliegenden verschiebbaren Spannleiste (22) Vorsprünge (24) angeordnet sind, die die Lage von Reihen von Plättchen (20) und von dazwischen sich ergebenden Zwischenräumen (21) definieren.

8. Schneidvorrichtung nach Anspruch 7, dadurch gekennzeichnet, dass die Grundplatte (13) Vorsprünge (26) aufweist, die in die Fläche, auf die die Eigenhaut (11) aufgelegt wird, hervorstehen.

9. Schneidvorrichtung nach Anspruch 8, dadurch gekennzeichnet, dass die Vorsprünge durch die Spitzen von in die Grundplatte (13) eingelassenen Nadeln (26) gebildet werden.

10. Schneidvorrichtung nach Anspruch 7, dadurch gekennzeichnet, dass eine auf den Rahmen (10') aufsetzbare und entlang der Zwischenräume (21) führbare und Messer (41) enthaltende Vorrichtung (40, 41) vorgesehen ist.

11. Schneidvorrichtung nach Anspruch 7, dadurch gekennzeichnet, dass die Plättchen (20) mit Bohrungen (50) versehen sind, durch die auf der Oberseite der Plättchen (20) über einen Transportaufsatzstecker zum Abheben der Eigenhautstückchen und zum Absetzen der Eigenhautstückchen Druck an den an der Unterseite der Plättchen (20) anliegenden Eigenhautstückchen (4) wirksam werden kann.

12. Transportaufsatz zur Durchführung des Verfahrens nach Anspruch 6, dadurch gekennzeichnet, dass er Röhrchen (52) aufweist, die in die Öffnungen (50) in den Plättchen (20) einsteckbar sind, dass ferner Mittel vorgesehen sind (53, 54), um zum Abheben der Eigenhautstückchen (4) in den Röhrchen Unterdruck und zum Absetzen der Eigenhautstückchen (4) Überdruck zu erzeugen.

13. Stanzvorrichtung zur Durchführung des Verfahrens nach einem der Ansprüche 1 bis 4, mit der Öffnungen (3) in die Fremdhaut (2) eingebracht werden, mit einer Stanzwerkzeuge (74-1 bis 74-5) für eine Reihe von Öffnungen tragenden Stanzplatte (76), die in gleichen Abständen die Öffnungen (3) in die Fremdhaut (2) einschneiden, dadurch gekennzeichnet, dass zwischen Führungsschienen (63) eine zur Aufnahme der Fremdhaut (2) geeignete Transportunterlage (64) einschiebbar ist, die von einer Transportwalze (68) weiterbewegt wird.

## Claims

1. A method of production of a transplant (100), in which small pieces of the patient's own skin (4) are prepared, are moved to a foreign skin (2) provided with openings (3) are and inserted in them, characterized in that into a transposer device (90) at a place defined in a system of coordinates by positioning means (92) there is inserted a holder device (10) in which at likewise defined places there are small pieces (4) of the patient's own skin upon which at any time within the holder device (10) there is mounted a transport part (20, 51) which exhibits means (52–54) which upon being raised take with them the respective small piece (4) of the patient's own skin and upon being lowered release the small piece of the patient's own skin, that further at another place within the system of coordinates defined by further positioning means (93) a bed (64) with foreign skin (2) arranged on it is inserted, in which – with respect to the bed (64) – at likewise defined places the openings (3) are cut, and that further a manipulator (94, 95) moves the transport parts (20, 51) with the small pieces (4) of the patient's own skin to the openings (3) in the foreign skin (2) and inserts into them the small pieces (4) of the patient's own skin.

2. A method as in claim 1, characterized in that as foreign skin the skin from a cadaver, synthetic skin-substitute foil or other skin-substitute preparations are used.

3. A method as in claim 1, characterized in that in the foreign skin slits (65) are cut for the discharge of secretion.

4. A method as in claim 1, characterized in that the small pieces (4) of the patient's own skin are made polygonal, in particular square or hexagonal.

5. A method as in claim 4, characterized in that for the production of small square pieces (4) of the patient's own skin small plates (20) made of square shape to correspond are clamped in rows in the holder device which is made as a frame (10), by suitable clamping means (22, 23) in such a way that between the rows gaps (21) form, that further the frame (10) with the small plates (20) clamped in it is mounted in a first position (fig. 3) on a bed (13) and firmly clamped, that after that the patient's own skin (11) is cut along the gaps (21), the frame (10) with the small plates (20) clamped in it in rows is thereupon removed from the said skin (11), twisted through 90° and mounted once more upon the patient's own skin and clamped firmly onto the bed, and after that the said skin is again cut along the gaps (21).

6. A method as in claim 5, characterized in that after the patient's own skin has been cut twice along the gaps (21) transport mountings (51) are mounted on the small plates (20), which together with the small plates (20) form the whole transport part.

7. A cutter device for the performance of the method as in claim 5, by which the patient's own skin (11) is cut into small pieces (4), characterized in that on a baseplate (13) clamping devices (14) for clamping firmly a frame (10) and positioning means (12) are provided in order to give the frame a defined position, that the frame (10) is arranged to be able to turn about an axis perpendicular to the baseplate (13), and that further along an inner edge of the frame (10) and along a displaceable clamping-bar (22) lying opposite this inner edge projections (24) are arranged, which define the positions of rows of little plates (20) and of gaps (21) which result between them.

8. A cutter device as in claim 7, characterized in that the baseplate (13) exhibits projections (26) which stand up in the area upon which the patient's own skin (11) is laid.

9. A cutter device as in claim 8, characterized in that the projections are formed by the tips of needles (26) let into the baseplate (13).

10. A cutter device as in claim 7, characterized in that a mechanism (40, 41) is provided which may be mounted upon the frame (10') and guided along the gaps (21) and contains knives (41).

11. A cutter device as in claim 7, characterized in that the small plates (20) are provided with drilled holes (50) through which upon the upper face of the respective small plate (20) via a transport-mounting plug pressure may for the raising of the small pieces of the patient's own skin and for setting down the small pieces of the patient's own skin, become effective against the small pieces (4) of the patient's own skin resting against the underside of the small plates (20).

12. A transport mounting for the performance of the method as in claim 6, characterized in that it exhibits small tubes (52) which may be plugged into the openings (50) in the small plates (20), that again means (53, 54) are provided for generating in the small tubes reduced pressure for the raising of the small pieces (4) of the patient's own skin and overpressure for the depositing of the small pieces (4) of the said skin.

13. A stamping mechanism for the performance of the method as in one of the claims 1 to 4, by which openings (3) are introduced into the foreign skin (2), having a stamping plate (76) which for a row of openings carries stamping tools (74-1 to 74-5) which cut the openings (3) in the foreign skin (2) at equal intervals, characterized in that between guiderails (63) a conveyor bed (64) may be inserted, which is suitable for receiving the foreign skin (2) and which is moved on by a conveyor roll (68).

**Revendications**

1. Procédé pour la fabrication d'un transplant (100), selon lequel de petits morceaux de peau propre (4) sont préparés, déplacés jusqu'à une peau étrangère (2) dans laquelle ont été prévues des ouvertures (3) et sont placés dans celles-ci, caractérisé en ce que dans une installation de transfert (90) est disposé à une position définie par un système de coordonnées, un dispositif de maintien (10) à l'aide de moyens de positionnement (92), dans laquelle se trouvent aussi à des positions définies des petits morceaux de peau propre (4) sur chacun desquels est déposé, à l'intérieur du dispositif de maintien, un organe de transport (20, 51) qui présente des moyens (52, 54) servant au soulèvement de chaque petit morceau de peau propre (4), au transport et au dépôt des petits morceaux de peau propre (4), et en ce que, en outre, une console (64), sur laquelle est posée une peau étrangère (2) dans laquelle sont découpées aussi des ouvertures (3) à des emplacements définis, est disposée à une autre position définie intérieurement au système de coordonnées, par un moyen supplémentaire de positionnement (93) – pour ce qui est de la console (64) – et en ce que, en outre, un manipulateur (94, 95) déplace l'organe de transport (20, 51) avec les petits morceaux de peau propre (4) jusqu'aux ouvertures (3) de la peau étrangère (2) et met les petits morceaux de peau propre (4) en place dans cette dernière.

2. Procédé selon la revendication 1, caractérisé en ce que l'on emploie comme peau étrangère la peau d'un cadavre, une feuille d'un substitut synthétique de peau ou d'autres préparations de substituts de peau.

3. Procédé selon la revendication 1, caractérisé en ce que dans la peau étrangère (2) sont découpées des fentes pour l'écoulement de sécrétions.

4. Procédé selon la revendication 1, caractérisé en ce que les petits morceaux de peau propre (4) sont à configuration polygonale, en particulier carrée ou hexagonale.

5. Procédé selon la revendication 4, caractérisé en ce que pour la fabrication des petits morceaux de peau propre (4) carrés, des petits plateaux (20) carrés correspondants disposés en rangée sont contraints par des moyens de contrainte (22, 23) appropriés dans un dispositif de maintien conformé en cadre (10); et en ce qu'ils limitent des intervalles (21) entre les rangées, et en ce qu'en outre le cadre (10) est disposé et fermement immobilisé sur une console (13) avec les petits plateaux (20) contraints dans une première position (fig. 3), et en ce qu'ensuite, la peau propre (11) est découpée le long des intervalles (21) puis le cadre (10) avec les petits plateaux (20) contraints en rangées est enlevé de la peau propre (11) tourné de 90° et à nouveau disposé sur la peau propre et fermement immobilisé sur la console, et ensuite la peau propre est à nouveau découpée le long des intervalles.

6. Procédé selon la revendication 5, caractérisé en ce qu'après la double coupe de la peau propre le long des intervalles (21), des tables de transport (51) sont placées sur les petits plateaux (20) et elles constituent avec les petits plateaux (20) l'ensemble de l'organe de transport.

7. Dispositif de coupe pour la mise en oeuvre du procédé selon la revendication 5 avec lequel on découpe la peau propre (11) en petits morceaux (4), caractérisé en ce que sont prévus sur une embase (13) des dispositifs de contrainte (14) pour contraindre un cadre (10) et des moyens de positionnement (12), pour donner au cadre (10) une position définie, et en ce que le cadre (10) est monté mobile autour d'un axe perpendiculaire à l'embase (13) et en ce qu'en outre sont disposés des saillies (24) le long d'un bord intérieur du cadre (10) et le long d'une barre de contrainte (23) coulissante opposée à ce bord intérieur, ces saillies définissant la position des rangées des petits plateaux (20) et les intervalles (21) en résultant entre ces derniers.

8. Dispositif de coupe selon la revendication 7, caractérisé en ce que l'embase (13) présente des saillies (26) qui sont en relief par rapport à la surface sur laquelle est posée la peau propre (11).

9. Dispositif de coupe selon la revendication 8, caractérisé en ce que les saillies sont constituées par des pointes d'aiguilles (26) noyées dans la console (13).

10. Dispositif de coupe selon la revendication 7, caractérisé en ce qu'est prévu un dispositif (40, 41) comprenant une lame (41) disposé sur le cadre (10) et pouvant être guidé le long des intervalles (21).

11. Dispositif de coupe selon la revendication 7, caractérisé en ce que les petits plateaux (20) sont prévus avec des trous (50) à travers lesquels une pression d'un organe de transport situé à la face supérieure de ces petits plateaux (20) peut agir sur les petits morceaux de peau propre (4) proches de la face inférieure desdits petits plateaux (20) pour le soulèvement des petits morceaux de peau propre (4) et pour le dépôt de ces petits morceaux de peau propre (4).

12. Table de transport pour la mise en oeuvre du procédé selon la revendication 6, caractérisé en ce qu'elle présente de petits tubes (52) qui peuvent être introduits dans les trous (50) des petits plateaux (20), et en ce que, en outre, des moyens (53, 54) sont prévus pour créer une dépression pour le soulèvement des petits morceaux de peau propre (4) et une surpression pour déposer les petits morceaux de peau propre (4).

13. Dispositif de perforation pour la mise en oeuvre du procédé selon l'une des revendications 1 à 4, avec les ouvertures (3) pratiquées dans la peau étrangère (2), avec un outil de perforation (74-1 à 74-5) portant un plateau de perforation (76) pour une rangée d'ouvertures qui découpe à des intervalles identiques les ouvertures (3) dans la peau étrangère, caractérisé en ce que, entre des rails de guidage (63) est montée coulissante pour la préhension de la peau étrangère (2) une console de transort (64) qui est déplacée de façon continue par un cylindre d'entraînement (68).

*Fig.1*

*Fig.2*

0156806

Fig.3

Fig.4

13

*Fig.5*

*Fig.6*

*Fig.7*

Fig.8

Fig.9

*Fig.10*

*Fig.11*

Fig.12